# EUROPÄISCHE PATENTSCHRIFT

(11) **EP 1 562 879 B1**
(45) Veröffentlichungstag und Bekanntmachung des Hinweises auf die Patenterteilung: **09.12.2009**
(21) Anmeldenummer: 03795802.2
(22) Anmeldetag: 28.10.2003
(51) Int. Cl.: C07C 5/333, C07C 11/06

(54) **VERFAHREN ZUR KATALYTISCHEN DEHYDRIERUNG VON KOHLENWASSERSTOFFEN**
METHOD FOR CATALYTICALLY DEHYDRATING HYDROCARBONS
PROCEDE DE DESHYDROGENATION CATALYTIQUE D'HYDROCARBURES

(30) Priorität: 31.10.2002 DE 10251135
(43) Veröffentlichungstag der Anmeldung: 17.08.2005
(73) Patentinhaber: Uhde GmbH, 44141 Dortmund (DE)
(72) Erfinder: THIAGARAJAN, Natarajan, 44141 Dortmund (DE); HEINRITZ-ADRIAN, Max, 48145 Münster (DE); WENZEL, Sascha, 44791 Bochum (DE); MENZEL, Johannes, 45731 Waltrop (DE)
(86) Internationale Anmeldenummer: PCT/EP2003/011948
(87) Internationale Veröffentlichungsnummer: WO 2004/039920

(56) Entgegenhaltungen:
- WO-A-96/19424
- US-A- 4 599 471
- US-A- 4 886 928

## Beschreibung

Bei der katalytischen Dehydrierung von Kohlenwasserstoffen nach der folgenden Formel:

CₙH₂ₙ₊₂ ↔ CₙH₂ₙ + H₂, (1)

die üblicherweise in der Gasphase bei einer Temperatur zwischen 540 °C und 820 °C durchgeführt wird, handelt es sich um eine stark endotherme Gleichgewichtsreaktion, deren Umsätze thermodynamisch begrenzt sind und von den jeweiligen Partialdrücken und der Temperatur abhängen. Die Dehydrierungsreaktion wird durch geringe Partialdrücke der Kohlenwasserstoffe und durch hohe Temperaturen begünstigt. In Nebenreaktionen bilden sich Crackprodukte, welche sich als Kohlenstoffablagerungen auf dem Katalysator ablagern und zur Deaktivierung des Katalysators führen, so dass der Katalysator in technischen Betrieb zyklisch regeneriert werden muss.

Wird die Dehydrierung in einem adiabat betriebenen Katalysatorbett durchgeführt, nimmt durch die endotherme Reaktion die Temperatur über die Länge des Katalysatorbettes ab. Der Umsatz im Katalysatorbett ist somit beschränkt, so dass für die gewünschten hohen Umsätze mehrere Katalysatorbetten notwendig sind und hinter jedem Katalysatorbett eine erneute Aufheizung stattfinden muss.

Die katalytische Dehydrierung von Paraffinen zu Olefinen kann aber auch in einem beheizten, bzw. isothermen Katalysatorbett durchgeführt werden. So wird z.B. in der US 5,235,121 ein Verfahren beschrieben, in dem ein Einsatzgemisch, welches aus leichten Paraffinen und Wasserdampf besteht, in einen Rohrreaktor gegeben wird, welcher von außen befeuert wird, d.h. das Katalysatorbett ist ein beheiztes Festbett. Der verwendete Katalysator ist derart beschaffen, dass sich in Gegenwart des Wasserdampfes kein Dampfreformierprozess, d.h. eine Reaktion der Kohlenwasserstoffe mit Wasserdampf unter Bildung von CO, CO₂ und H₂, einstellen kann. Der Katalysator wird zyklisch regeneriert. Ein ähnliches Verfahren wird auch in der DE 198 58 747 A1 beschrieben.

Durch die Beheizung des Katalysatorbettes bzw. die isotherme Fahrweise können in einer Katalysatorschüttung sehr hohe Umsätze erreicht werden. Nachteilig ist jedoch, dass solche sehr hohen Umsätze bedingt durch die Lage des thermodynamischen Gleichgewichts nur bei hohen Temperaturen erreicht werden können, wodurch die Selektivität der Olefinbildung verringert wird.

Vorteilhaft an der beschriebenen Fahrweise in Gegenwart von Wasserdampf ist, dass durch den Wasserdampf der Partialdruck der Kohlenwasserstoffe reduziert und somit der Umsatz erhöht wird. Weiterhin wird durch den Dampfeinsatz ein Teil der Kohlenstoffablagerungen auf dem Katalysator zu CO₂ umgesetzt und die Zykluszeiten, d.h. Betriebszeiten der Dehydrierung zwischen den Regenerierungen, können verlängert werden. Allerdings ist die Zugabe zu großer Mengen an Dampf nachteilig, da es zu einer großen Volumenzunahme des Gasstromes kommt, was zu zusätzlichen Investitionskosten führt und die Wirtschaftlichkeit des Verfahrens belastet. Weiterhin nimmt dann auch die Gefahr der Dampfreformierung von Kohlenwasserstoffen zu, was Produktverlust bzw. Ausbeuteverringerung zur Folge hat. Die Menge an Dampf, die zugegeben werden kann, ohne dass die beschriebenen Probleme auftauchen, ist von dem absoluten Druck, bei dem die Reaktion gefahren wird, sowie vom verwendeten Dehydrierungskatalysator abhängig.

Eine weitere Möglichkeit, die thermodynamische Limitierung des Gleichgewichtsumsatzes zu überwinden, besteht darin, dass durch die Zufuhr von Sauerstoff ein Teil des in der Dehydrierung entstehenden Wasserstoffs gemäß

2 H₂ + O₂ → 2 H₂O , (2)

selektiv verbrannt wird - als Bezeichnung hierfür wird auch das Kürzel "SHC" für "Selective Hydrogen Combustion" verwendet - und damit das Gleichgewicht der Dehydrierung in Richtung höherer Umsätze verschoben wird. Beispielsweise beschreibt die EP 0 799 169 B1 einen Reaktor für ein solches Dehydrier-Verfahren mit SHC, bei dem ein Paraffin-Sauerstoff-Gemisch über einen ersten Katalysator geleitet wird, der sowohl dehydriert als auch entstandenen Wasserstoff selektiv oxidiert, einer weiteren Zugabe von Sauerstoff in einen Zwischenraum des Reaktors und einem anschließenden zweiten Katalysator, welcher ebenfalls sowohl dehydriert als auch entstandenen Wasserstoff selektiv oxidiert. Der Prozess der EP 0 799 169 B1 wird autotherm geführt, die stark exotherme Reaktion (2) des Wasserstoffs mit dem Sauerstoff liefert die Energie zur Durchführung der endothermen Dehydrierungs-Reaktion (1).

Weiterhin beschreibt z.B. die WO 96/33150 ein Verfahren, bei dem das Paraffingemisch zunächst in einer ersten Stufe dehydriert wird, daraufhin wird Sauerstoff hinzugegeben, wobei in mindestens einer zweiten Stufe dieser Sauerstoff mit dem bei der Dehydrierung freigesetzten Wasserstoff zu Wasserdampf reagiert. Wenigstens ein Teilstrom des erhaltenen Produkts wird einer Nach-Dehydrierung unterworfen, um nicht umgesetzte Paraffine noch zu dehydrieren, auch eine Rückführung eines Teilstroms in die erste Stufe wird vorgeschlagen.

Nachteilig ist bei diesen beiden Verfahren, dass durch die Zugabe des Sauerstoffs und die exotherme Wasserstoff-Oxidation das Reaktionsgas auf sehr hohe Temperaturen aufgeheizt wird, wodurch die Selektivität der nachfolgenden katalytischen Dehydrierung verringert wird. Dies ist insbesondere für isotherm geführte Dehydrierungen in der ersten Stufe der Fall, da bei adiabaten Dehydrierungen in der ersten Stufe durch den Temperaturabfall im Katalysatorbett eine geringere Eingangstemperatur zur SHC-Stufe vorliegt als ber der isothermen Dehydrierung.

Das Problem der Überhitzung durch die Wasserstoff-Oxidation kann dadurch gelöst werden, dass durch eine Zwischenkühlung vor der selektiven Wasserstoff-Oxidation die Eingangstemperatur zum zweiten Katalysatorbett abgesenkt wird. In der US 4,599,471 wird z.B. eine solche Zwischenkühlung vorgeschlagen, die entweder indirekt oder direkt ausgeführt werden kann. Die direkte Kühlung kann durch Inertgase wie Stickstoff, Helium etc. oder durch Dampf durchgeführt werden.

Die Temperatureinstellung durch indirekte Kühlung ist jedoch nachteilig, weil sie feste Wärmetauscher-Installationen erfordert, was keine gezielte Temperaturführung bei der Regeneration des Katalysatorbettes ermöglicht, oder aber eine Installation für eine zeitweilige Auskopplung des Wärmetauschers erforderlich macht, etwa einen durch zusätzliche Armaturen wie Ventile abgesperrbaren Bypass. Letzteres wäre angesichts der großen Rohrquerschnitte und der hohen Betriebstemperaturen von ca. 500 - 650 °C, die die Dehydrierung mit sich bringt, mit extrem hohem Aufwand verbunden.

Die direkte Kühlung durch Inertgase ist nachteilig, da diese in der späteren Produktaufbereitung durch aufwendige Prozessstufen vom Produkt abgetrennt werden müssen. Die direkte Kühlung mit Dampf ist nachteilig, da Dampf in der Reaktion, wie oben beschrieben, nicht inert ist und sich durch die Kühlung ein bestimmtes Dampf-zu-Kohlenwasserstoff-Verhältnis in Abhängigkeit der Kühlung einstellt. Dadurch erhöht sich bei starker Kühlung die Dampfmenge erheblich, was sich nachteilig auf den Prozess auswirkt, indem das erforderliche Bauvolumen und die Wahrscheinlichkeit der unerwünschten Dampfreformierung von Kohlenwasserstoffen zunehmen.

Die Aufgabe der Erfindung ist es daher, eine Verfahrensführung zur Dehydrierung mit anschließender Wasserstoff-Oxidation und weiterer Dehydrierung zur Verfügung zu stellen, welche vor der Wasserstoff-Oxidation eine direkte Kühlung des Zwischenstroms ermöglicht, wobei zum Einen eine einfache Abtrennung des Kühlmediums bei der Produktaufbereitung möglich ist und zum Anderen das Kühlmedium keinen nachteiligen Einfluss auf die nachfolgende Dehydrierung nimmt.

Die Erfindung löst die Aufgabe gemäß dem Hauptanspruch, indem
- in einer ersten Stufe ein Kohlenwasserstoff, insbesondere Alkane enthaltendes Gemisch, welches Wasserdampf aufweisen kann und im wesentlichen keinen Sauerstoff aufweist, in kontinuierlicher Fahrweise durch ein erstes Katalysatorbett geleitet wird, welches übliche Dehydrierungsbedingungen aufweist,
- nachfolgend dem aus der ersten Stufe erhaltenen Reaktionsgemisch, bestehend aus Alkanen, Alkenen, Wasserdampf, Wasserstoff und Nebenprodukten, sowohl flüssiges Wasser als auch Wasserdampf und ein Sauerstoff enthaltendes Gas beigemischt werden, und
- nachfolgend das erhaltene Reaktionsgemisch in kontinuierlicher Fahrweise in einer zweiten Stufe durch ein weiteres Katalysatorbett zur Oxidation von Wasserstoff und der weiteren Dehydrierung von Kohlenwasserstoffen geleitet wird.

In Ausgestaltung der Erfindung wird das erste Katalysatorbett beheizt und bevorzugt in einer annähernd isothermen Fahrweise betrieben, wobei dessen apparativer Aufbau dem der US 5,235,121 angelehnt sein kann.

Die Zugabe von sowohl flüssigem Wasser als auch Wasserdampf vor der selektiven Wasserstoff-Oxidation erlaubt es, gleichzeitig und unabhängig die optimale Temperatur und ein gezieltes Verhältnis von H₂O zu Kohlenwasserstoffen in der Gasphase einstellen zu können, wobei die Verdampfungswärme des eingebrachten flüssigen Wassers, welches vollständig verdampft, für die Temperatureinstellung eine wesentliche Rolle spielt. Wasserdampf kann durch Kondensation in späteren Prozessstufen leicht abgetrennt werden. Durch die gezielte Einstellung des Verhältnisses von H₂O zu Kohlenwasserstoffen in der Gasphase wird eine prozessbenachteiligende Dampfmenge vermieden, insbesondere eine Dampfreformierung der Kohlenwasserstoffe oder ein zu großer Gasvolumenstrom durch zuviel Dampf, oder aufgrund zu wenig Dampf zu hohe Kohlenstoffablagerung auf dem Katalysator oder zu hohe Kohlenwasserstoff-Partialdrücke im reagierenden Gas.

In einer weiteren Ausgestaltung der Erfindung wird nachfolgend dem aus der zweiten Stufe erhaltenen Reaktionsgemisch ein Sauerstoff enthaltendes Gas beigemischt und das erhaltene Reaktionsgemisch in kontinuierlicher Fahrweise in mindestens einer dritten Stufe durch ein weiteres Katalysatorbett geleitet, wobei das Katalysatorbett der dritten Stufe ein ähnliches oder das gleiche Katalysatormaterial wie die vorangegangenen Stufen haben kann. Dies ermöglicht eine gestufte Zugabe des Sauerstoffs in die einzelnen Stufen, um die produktschädliche Sauerstoffkonzentration gering zu halten

Als Katalysatoren wird in Ausgestaltung der Erfindung für das Katalysatorbett der ersten Stufe ein beliebiger, üblicher Dehydrierungskatalysatoren verwendet. Für das zweite und ggf. weitere Katalysatorbetten sind solche Dehydrierungskatalysatoren zu verwenden, welche sich nicht nur durch Aktivität zur Dehydrierung auszeichnen, sondern auch Aktivität zur SHC haben.

Dies sind z.B. Edelmetall-haltige Katalysatoren auf entsprechenden Trägermaterialien, wie z.B. Al₂O₃, SiO₂, ZrO₂, Zeolithe, Hydrotalcit, CaAl₂O₄, ZnAl₂O₄, MgAl₂O4₃ oder deren Gemische. Diese können mit weiteren Komponenten wie z.B. Elementen der Gruppe IV A des Periodensystems, oder auch der Gruppe I A dotiert sein. Aber auch Übergangsmetalloxid-basierte Dehydrierungskatalysatoren wie z.B. Katalysatoren auf Basis Fe₂O₃, Cr₂O₃, MoO₃ oder V₂O₅ sind im Sinne der Erfindung einsetzbar.

In einer Ausgestaltung der Erfindung wird für jede beliebige der Stufen, insbesondere für die zweite und ggf. weitere Stufen, ein Katalysator eingesetzt, welcher Pt und Sn enthält, welches sich auf einem Träger befindet, welcher im Wesentlichen Aluminat, insbesondere Zn-Aluminat enthält.

In einer weiteren Ausgestaltung der Erfindung wird als Katalysator im Katalysatorbett der zweiten und ggf. weiterer Stufen ein gesonderter Katalysator zur Wasserstoff-Oxidation, der eine bessere Selektivität als die der Dehydrierungskatalysatoren bei der Wasserstoff-Oxidation aufweist, zusammen mit dem Dehydrierungskatalysator eingesetzt.

In einer weiteren Ausgestaltung der Erfindung wird das Reaktionsgemisch nach der zweiten Stufe gekühlt, bevor es zu einem weiteren Katalysatorbett in einer dritten Stufe geleitet wird. Beim Einsatz weiterer Stufen kann eine solche Zwischenkühlung auch zwischen je zwei Stufen erfolgen. Die Kühlung kann auch indirekt stattfinden und die entnommene Wärme kann zur Aufheizung des Einsatzgemisches vor der ersten Stufe genutzt werden. Dementsprechend sind auch Wasser und Wasserdampf zuzudosieren, je nach Bedarf. Eine Kühlung vor der zweiten und weiteren Stufen hat den Vorteil, dass die Reaktionsgeschwindigkeit von Nebenreaktionen bei der Zugabe von Sauerstoff verringert wird. Somit wird eine Steigerung der Selektivität erreicht.

In einer weiteren Ausgestaltung der Erfindung wird als Sauerstoff enthaltendes Gas sauerstoffangereicherte Luft, z.B. mit einem Sauerstoffgehalt von 90 bis 95 %, eingesetzt. Hierdurch wird erreicht, dass die verwendeten Apparate kleiner baubar sind im Vergleich zu einer Zugabe von Luft. Eine Zugabe von Luft hat dagegen den Vorteil, dass die Partialdrücke der Kohlenwasserstoffe sinken und der Gleichgewichtsumsatz gesteigert werden kann, Luft ist auch preiswerter.

In einer weiteren Ausgestaltung der Erfindung wird die Menge des zudosierten, Sauerstoff enthaltenden Gases in die zweite oder weitere Stufen über die gemessenen Austrittstemperaturen der jeweils vorgeschalteten Katalysatorbetten oder über die Austrittstemperatur des letzten Katalysatorbetts geregelt.

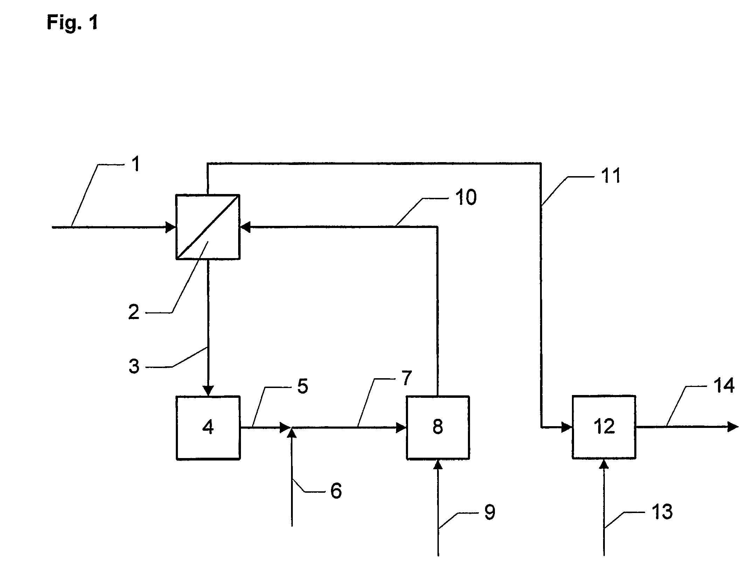

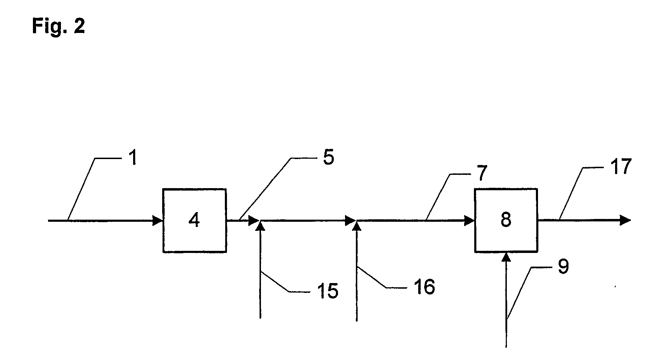

Die Erfindung wird im Folgenden anhand von 2 Beispielen näher erläutert. Fig. 1 zeigt das erfindungsgemäße Verfahren in dreistufiger Ausführung mit Zwischenkühlung, Fig. 2 zeigt das erfindungsgemäße Verfahren in zweistufiger Ausführung.

Fig. 1: Das Einsatzgemisch 1, bestehend aus Propan und Wasserdampf, wird in dem Gas-Gas-Wärmetauscher 2 auf 550 °C aufgeheizt. Das aufgeheizte Einsatzgemisch 3 wird in die Dehydrierstufe 4 geleitet, wo ein Teil des Propans zu Propylen reagiert. Durch Beheizung wird am Austritt der Dehydrierstufe 4 eine Temperatur von 570 °C eingestellt. Dem Reaktionsgemisch 5 wird ein definiertes Wasser-Wasserdampf-Gemisch 6 zugegeben, wodurch sich das Gemisch 7 auf 510 °C abkühlt und das Wasserdampf zu Kohlenwasserstoff Verhältnis in der Dehydrierstufe 8 gezielt eingestellt wird.

Das Gemisch 7 wird in die Dehydrierstufe 8 geleitet, unmittelbar vor dem Eintritt in dessen Katalysatorbett wird sauerstoffangereicherte Luft 9 zudosiert. Das erhaltene Produktgemisch 10 weist eine Temperatur von 590 °C auf und gibt einen Teil seiner Wärme im Gas-Gas-Wärmetauscher 2 an das Einsatzgemisch 1 ab. Das Produktgemisch 11 verlässt den Wärmetauscher 2 mit 510 °C und wird in die Dehydrierstufe 12 geleitet, unmittelbar vor dem Eintritt in dessen Katalysatorbett wird sauerstoffangereicherte Luft 13 zudosiert. Das erhaltene Produktgas 14 wird zur Aufbereitung in nachfolgende Einheiten weitergeleitet. Als Katalysator wird in allen Dehydrierstufen üblicher Dehydrierungskatalysator verwendet.

Fig. 2: Das Einsatzgemisch 1, bestehend aus Propan und Wasserdampf, wird in die Dehydrierstufe 4 geleitet, wo ein Teil des Propans zu Propylen reagiert. Dem Reaktionsgemisch 5 wird ein flüssiges Wasser 15 und Wasserdampf 16 zugegeben, wodurch das Wasserdampf-zu-Kohlenwasserstoff-Verhältnis in der Dehydrierstufe 8 gezielt eingestellt wird. Das Gemisch 7 wird in die Dehydrierstufe 8 geleitet, unmittelbar vor dem Eintritt in dessen Katalysatorbett wird sauerstoffangereicherte Luft 9 zudosiert. Das erhaltene Produktgemisch 10 wird zur Aufbereitung in nachfolgende Einheiten weitergeleitet. Als Katalysator wird in beiden Dehydrierstufen üblicher Dehydrierungskatalysator verwendet.

Zur weiteren Veranschaulichung des Verfahrens in der Ausgestaltung von Fig. 2 dienen 2 Rechenbeispiele, wobei Gasbestandteile aus Nebenreaktionen oder Verunreinigungen, wie z.B. CO₂, CH₄, N₂, als "Übrige" bezeichnet werden.

Rechenbeispiel 1

| Strom Nr. | | 1 | 5 | 7 | 9 | 15 | 16 | 17 |
|---|---|---|---|---|---|---|---|---|
| Temperatur | [° C] | 550 | 580 | 532 | 180 | 180 | 240 | 580 |
| Druck | [bar] | 9 | 6 | 5,9 | 8 | 12 | 12 | 5 |
| Sauerstoff O₂ | [kg/h] | | | | 7344 | | | |
| Wasserstoff H₂ | [kg/h] | | 2514 | 2514 | | | | 3439 |
| Dampf H₂O | [kg/h] | 176845 | 175017 | 210387 | | | 26730 | 215635 |
| Wasser H₂O | [kg/h] | | | | | 8640 | | |
| Propan C₃H₈ | [kg/h] | 123511 | 86458 | 86458 | | | | 74106 |
| Übrige | [kg/h] | 2470 | 4883 | 4883 | 714 | | | 9687 |
| Propen C₃H₆ | [kg/h] | | 33954 | 33954 | | | | 43387 |

Rechenbeispiel 2

| Strom Nr. | | 1 | 5 | 7 | 9 | 15 | 16 | 17 |
|---|---|---|---|---|---|---|---|---|
| Temperatur | [° C] | 550 | 575 | 486 | 180 | 115 | 180 | 600 |
| Druck | [bar] | 6 | 2 | 1,9 | 12 | 4 | 4 | 1,1 |
| Sauerstoff O₂ | [kg/h] | | | | 10098 | | | |
| Wasserstoff H₂ | [kg/h] | | 3306 | 3306 | | | | 5271 |
| Dampf H₂O | [kg/h] | 50527 | 47628 | 67839 | | | 4010 | 73089 |
| Wasser H₂O | [kg/h] | | | | | 16201 | | |
| Propan C₃H₈ | [kg/h] | 123511 | 80282 | 80282 | | | | 65461 |
| Übrige | [kg/h] | 2470 | 6091 | 6091 | 465 | | | 13591 |
| Propen C₃H₆ | [kg/h] | | 39201 | 39201 | | | | 49870 |

### Bezugszeichenliste

- 1: Einsatzgemisch
- 2: Gas-Gas-Wärmetauscher
- 3: aufgeheiztes Einsatzgemisch
- 4: Dehydrierstufe
- 5: Reaktionsgemisch
- 6: Wasser-Wasserdampf-Gemisch
- 7: Gemisch
- 8: Dehydrierstufe
- 9: sauerstoffangereicherte Luft
- 10: Produktgemisch
- 11: Produktgemisch
- 12: Dehydrierstufe
- 13: sauerstoffangereicherte Luft
- 14: Produktgas
- 15: Wasser
- 16: Wasserdampf
- 17: Produktgemisch

## Patentansprüche

1. Verfahren zur Herstellung von ungesättigten Kohlenwasserstoffen,
• bei dem in einer ersten Stufe ein Kohlenwasserstoff, insbesondere Alkane enthaltendes Gemisch, welches Wasserdampf aufweisen kann und im wesentlichen keinen Sauerstoff aufweist, in kontinuierlicher Fahrweise durch ein erstes Katalysatorbett geleitet wird, welches übliche Dehydrierungsbedingungen aufweist,
• nachfolgend dem aus der ersten Stufe erhaltenen Reaktionsgemisch sowohl flüssiges Wasser als auch Wasserdampf und ein Sauerstoff enthaltendes Gas beigemischt werden, und
• nachfolgend das erhaltene Reaktionsgemisch in kontinuierlicher Fahrweise in einer zweiten Stufe durch ein weiteres Katalysatorbett zur Oxidation von Wasserstoff und der weiteren Dehydrierung von Kohlenwasserstoffen geleitet wird.

2. Verfahren nach Anspruch 1, **dadurch gekennzeichnet, dass** die das erste Katalysatorbett beheizt wird und die Beheizung der ersten Stufe bevorzugt so eingestellt wird, dass sich eine im wesentlichen isotherme Betriebsweise ergibt.

3. Verfahren nach einem der Ansprüche 1 oder 2, **dadurch gekennzeichnet, dass** nachfolgend dem aus der zweiten Stufe erhaltenen Reaktionsgemisch ein Sauerstoff enthaltendes Gas beigemischt wird und das erhaltene Reaktionsgemisch in kontinuierlicher Fahrweise in mindestens einer dritten Stufe durch ein weiteres Katalysatorbett geleitet wird.

4. Verfahren nach einem der Ansprüche 1 bis 3, **dadurch gekennzeichnet, dass** das Reaktionsgemisch nach der zweiten Stufe durch eine Kühlungseinrichtung gekühlt wird, bevor es zu einem weiteren Katalysatorbett in einer dritten Stufe geleitet wird.

5. Verfahren nach einem der Ansprüche 1 bis 4, **dadurch gekennzeichnet, dass** als Katalysatoren für das Katalysatorbett der ersten Stufe beliebige, übliche Dehydrierungskatalysatoren verwendet werden und für das zweite und ggf. weitere Katalysatorbetten solche Dehydrierungskatalysatoren verwendet werden, welche sich nicht nur durch Aktivität zur Dehydrierung auszeichnen, sondern auch Aktivität zur SHC haben.

6. Verfahren nach einem der Ansprüche 1 bis 5, **dadurch gekennzeichnet, dass** für jede beliebige der Stufen, insbesondere für die zweite und ggf. weitere Stufen, ein Katalysator eingesetzt wird, welcher Pt und Sn enthält, welches sich auf einem Träger befindet, welcher im Wesentlichen Aluminat, insbesondere Zn-Aluminat enthält.

7. Verfahren nach einem der Ansprüche 1 bis 6, **dadurch gekennzeichnet, dass** in den der zweiten oder weiteren Stufen ein spezieller Katalysator zur Wasserstoff-Oxidation, der eine bessere Selektivität als übliche Dehydrierungskatalysatoren bezüglich der Wasserstoff-Oxidation aufweist, alleine oder zusammen mit einem üblichen Dehydrierungskatalysator eingesetzt wird.

8. Verfahren nach einem der Ansprüche 1 bis 7, **dadurch gekennzeichnet, dass** das Sauerstoff enthaltende Gas sauerstoffangereicherte Luft ist.

9. Verfahren nach einem der vorangegangenen Ansprüche 1 bis 8, **dadurch gekennzeichnet, dass** die Menge des zudosierten, Sauerstoff enthaltenden Gases in die zweite oder weiteren Stufen über die gemessenen Austrittstemperaturen der jeweils vorgeschalteten Katalysatorbetten oder über die Austrittstemperatur des letzten Katalysatorbetts geregelt wird.

## Claims

1. Process for the production of unsaturated hydrocarbons, with the following layout:
• In a first step hydrocarbon, in particular a mixture containing alkanes, which may have a water vapour content but which is essentially free from oxygen, penetrates in a continuous stream through a first catalyst bed, the latter exhibiting the standard dehydration conditions;
• subsequently the reaction mixture obtained in the first step is mixed with liquid phase water and water vapour as well as with an oxygen-bearing gas;
• and then the reaction mixture is fed in a continuous stream to a further catalyst bed as second step in which hydrogen oxidation and further dehydration of the hydrocarbons take place.

2. Process in accordance with Claim 1,
**characterised in that** the first catalyst bed is heated and the heating of the first step is preferably adjusted in such a manner that an essentially isothermal operating mode is obtained.

3. Process in accordance with any one of the preceding Claims 1 or 2,
**characterised in that** oxygen-bearing gas is added to the reaction mixture produced in the second step and the reaction mixture thus obtained flows in a continuous stream through a further catalyst bed in at least a third process step.

4. Process in accordance with any one of the preceding Claims 1 to 3,
**characterised in that** the reaction mixture is cooled in a cooling unit downstream of the second step prior to entering a further catalyst bed in a third process step.

5. Process in accordance with any one of the preceding Claims 1 to 4,
**characterised in that** the catalyst bed of the first step uses any standard commercial dehydration catalyst and the second and any further catalyst bed are provided with dehydration catalysts that exhibit not only dehydration activity but also SHC activity (selective hydration combustion).

6. Process in accordance with any one of the preceding Claims 1 to 5,
**characterised in that** any of the process steps, particularly the second and further steps, is equipped with a catalyst that contains Pt and Sn applied to a carrier element essentially contains aluminate, in particular Zn aluminate.

7. Process in accordance with any one of the preceding Claims 1 to 6,
**characterised in that** the second and any further step are provided with a specialist catalyst for hydrogen oxidation, hence a catalyst that improves the selectivity compared to that of standard dehydration catalysts when it comes to hydrogen oxidation, the said specialist catalysts being employed as singleton or in combination with standard dehydration catalysts.

8. Process in accordance with any one of the preceding Claims 1 to 7,
**characterised in that** the oxygen-bearing gas is oxygen-rich air.

9. Process in accordance with any one of the preceding Claims 1 to 8,
**characterised in that** the quantity of oxygen-bearing gas added in the second or further steps is controlled via the temperature measured at the outlet of the respective upstream catalyst bed or via the outlet temperature of the last catalyst bed.

## Revendications

1. Procédé pour la production d'hydrocarbures non saturés, avec la configuration décrite ci-après :
• Dans une première étape, l'hydrocarbure, en particulier des alcanes ayant une teneur en vapeur d'eau mais qui sont essentiellement libres d'oxygène, s'écoule en un mode continu dans un premier lit catalytique, ce dernier fonctionnant à des conditions de déshydratation standardisées ;
• le mélange de réaction obtenu dans la première étape est mélangé avec de l'eau en phase liquide et de la vapeur d'eau, pour ajouter ensuite du gaz oxygéné ;
• et après, le mélange de réaction est envoyé en courant continu vers un autre lit catalytique de la deuxième étape, pour faire oxyder de l'hydrogène, c.-à-d l'oxydation de l'hydrogène et l'hydratation ultérieure des hydrocarbures ont lieu dans la dite deuxième étape.

2. Procédé selon la revendication No.1,
**caractérisé en ce que** le premier lit catalytique est chauffé et que le chauffage de la première étape est, de préférence, commandé de telle manière que le régime soit essentiellement isothermique.

3. Procédé selon l'une des revendications précédentes, Nos. 1 ou 2,
**caractérisé en ce que** du gaz oxygéné est alors ajouté au mélange de réaction obtenu dans la deuxième étape et que le mélange de réaction ainsi obtenu s'écoule en un mode continu vers un autre lit catalytique disposé dans une troisième étape au moins requise dans ce procédé.

4. Procédé selon l'une des revendications précédentes, Nos. 1 à 3,
**caractérisé en ce que** le mélange de réaction subit un refroidissement dans une unité de refroidissement disposée en aval de la deuxième étape, avant l'entrée du dit mélange dans un autre lit catalytique de la troisième étape.

5. Procédé selon l'une des revendications précédentes, Nos. 1 à 4,
**caractérisé en ce que** les catalyseurs utilisés pour le lit catalytique de la première étape sont du type standard pour la déshydratation et que ceux mis en oeuvre pour le deuxième lit catalytique et d'autres encore, s'il y en a, sont du type de bonne activité de déshydratation et, de plus, d'une activité adéquate SHC (selective hydration combustion).

6. Procédé selon l'une des revendications précédentes, Nos.1 à 5,
**caractérisé en ce que** chaque catalyseur mis en oeuvre pour n'importe quelle étape de procédé, en particulier pour la deuxième étape et d'autres encore, s'il y en a, doit avoir une teneur en Pt et Sn et doit être appliqué sur un élément porteur approprié, ce dernier consistant essentiellement en un aluminate, en particulier d'un aluminate Zn.

7. Procédé selon l'une des revendications précédentes, Nos. 1 à 6,
**caractérisé en ce que** la deuxième étape et toute autre étape éventuellement présente sont dotées d'un catalyseur spécifique pour l'oxydation de l'hydrogène, donc un catalyseur qui améliore la sélectivité par rapport à un type standard de déshydratation concernant l'oxydation de l'hydrogène, et que le dit catalyseur est utilisé individuellement ou bien combiné avec un catalyseur standard de déshydratation.

8. Procédé selon l'une des revendications précédentes, Nos. 1 à 7,
**caractérisé en ce que** le gaz oxygéné est de l'air riche en oxygène.

9. Procédé selon l'une des revendications précédentes, Nos. 1 à 8,
**caractérisé en ce que** le débit de gaz oxygéné, qui est ajouté dans la deuxième étape ou toute autre étape éventuellement présente, est réglé sur la base de la température mesurée à la sortie du lit catalytique respectif disposé en amont ou bien sur la base de la température de sortie du dernier lit catalytique.
